# FASCICULE DE BREVET EUROPEEN

(11) **EP 0 884 295 B1**
(45) Date de publication et mention de la délivrance du brevet: **06.02.2002**
(21) Numéro de dépôt: 98201757.6
(22) Date de dépôt: 27.05.1998
(51) Int. Cl.: C07C 17/278, C07C 19/01

(54) **Procédé de préparation d'hydrocarbures halogénés**
Verfahren zur Herstellung von halogenierten Kohlenwasserstoffen
Process for the preparation of halogenated hydrocarbons

(30) Priorité: 02.06.1997 BE 9700476
(43) Date de publication de la demande: 16.12.1998
(73) Titulaire: SOLVAY, 1050 Bruxelles (BE)
(72) Inventeur: Schoebrechts, Jean-Paul, 5980 Grez-Doiceau (BE)
(74) Mandataire: Jacques, Philippe

(56) Documents cités:
- EP-A- 0 142 041
- EP-A- 0 729 932
- WO-A-97/15540

## Description

La présente invention concerne un procédé de préparation d'hydrocarbures halogénés comprenant au moins 3 atomes de carbone par réaction catalytique entre un haloalcane et une oléfine halogénée.

L'addition d'un haloalcane sur une oléfine halogénée est une réaction bien connue. Cependant, il est parfois difficile de contrôler la réaction de telle sorte qu'une seule molécule d'oléfine halogénée s'additionne à une molécule d'haloalcane (formation d'un produit d'addition ou adduit 1:1).

Très souvent, des dérivés du cuivre sont utilisés pour catalyser cette réaction d'addition. Par exemple, M. Asscher et D. Vofsi (J. Chem. Soc. 1887-1896, 1963) décrivent l'addition de tétrachlorure de carbone à des oléfines en présence de catalyseurs contenant du cuivre ou du fer. Toutefois, ce procédé présente l'inconvénient de nécessiter de longues périodes de chauffage pour obtenir le produit d'addition avec un rendement acceptable. La demande de brevet WO-A-97/15540 décrit en particulier la synthèse de 1,1,1,3,3-pentachloropropane par réaction de tétrachlorométhane avec du chlorure de vinyle en présence de chlorure de cuivre (I) et d'acétonitrile.

L'invention vise dès lors à fournir un procédé qui permet d'accéder aux hydrocarbures halogénés comprenant au moins 3 atomes de carbone en une seule étape et aux dépens de réactifs facilement accessibles avec d'excellents rendements.

En conséquence, la présente invention concerne la préparation d'hydrocarbures halogénés comprenant au moins 3 atomes de carbone par réaction entre un haloalcane choisi parmi les composés organiques saturés contenant de un à trois atomes de carbone et au moins 2 atomes de chlore et une oléfine halogénée en présence d'un composé organique du nickel comme catalyseur.

Le composé organique du nickel utilisé comme catalyseur dans le procédé selon la présente invention est choisi parmi les sels de nickel (II) formés avec les acides carboxyliques tels que les acides formique, acétique, acétylacétique, benzoïque, et parmi les organométalliques dans lesquels le nickel est lié à un atome de carbone aliphatique ou aromatique tels que les alkylnickel ou les arylnickel, le nickel tétracarbonyle. Le composé organique du nickel peut aussi être choisi parmi les complexes formés avec des ligands neutres comme les phosphines. Le composé organique du nickel utilisé comme catalyseur selon la présente invention peut être fixé sur un support polymérique, comme les polysiloxanes ou faire partie d'une structure dendrimérique formée au départ de silanes.

Avantageusement, le composé organique du nickel est un composé organométallique dans lequel l'atome de nickel est lié à un atome de carbone aromatique. De préférence, le composé organique du nickel est un composé de formule générale [Ni^{II}{4-RC₆H₂(CH₂NMe₂)₂-2,6}X] (appelée formule (I) dans ce qui suit) dans lequel R représente un atome d'hydrogène ou un groupement électrodonneur tel qu'une fonction amine, alkylamine, dialkylamine, une fonction alcool ou un groupe alkoxy, et X représente un ion halogénure. Les composés organiques du nickel de formule générale (I) peuvent être obtenus comme décrit dans Inorg. Chem. **1991,** 30, 3059-3068.

D'une manière préférée, dans la formule générale (I) ci-dessus, R représente un atome d'hydrogène ou un groupement amino (NH₂) et X représente un ion bromure.

Les hydrocarbures halogénés obtenus selon le procédé de la présente invention appartiennent, en général, à la famille des chloropropanes, des chlorobutanes ou des chloropentanes. Les atomes de carbone desdits chloropropanes, chlorobutanes et chloropentanes peuvent également être substitués par d'autres groupes fonctionnels tels que d'autres atomes d'halogène (comme des atomes de brome ou d'iode), des groupes alkyle ou halogénoalkyle, des groupes nitrile (CN) ou acide carboxylique (COOH). Les chloropropanes et les chlorobutanes sont préférés.

De préférence, les hydrocarbures halogénés obtenus selon le procédé de la présente invention répondent à la formule générale CₙH₍₂ₙ₊₂₎₋ₚClₚ dans laquelle n est un nombre entier et possède les valeurs 3 ou 4, p est un nombre entier qui possède les valeurs 3 à 7. Des exemples de composés obtenus selon le procédé de la présente invention sont le 1,1,1,3,3-pentachloropropane, le 1,1,1,3,3-pentachlorobutane, le 1,1,1,3-tétrachloropropane, le 1,1,3,3-tétrachlorobutane, le 1,1,1,3,3,3-hexachlororopropane et le 1,1-dichloro-2-trichlorométhylpropane. Parmi ces composés, le 1,1,1,3,3-pentachloropropane, le 1,1,1,3,3-pentachlorobutane et le 1,1-dichloro-2-trichlorométhylpropane sont préférés. Le 1,1,1,3,3-pentachlorobutane et le 1,1,1,3,3-pentachloropropane sont tout particulièrement préférés.

Les haloalcanes engagés dans le procédé selon la présente invention sont en général des composés organiques saturés. Ils peuvent également comprendre d'autres substituants tels que d'autres atomes d'halogène, des groupes alkyle ou halogénoalkyle. A titre d'exemples d'haloalcanes selon la présente invention, on peut citer le dichlorométhane, le chloroforme, le tétrachlorure de carbone, le 1,1,1-trichloroéthane, le 1,1,2-trichloro-1,2,2-trifluoroéthane. Le tétrachlorure de carbone est tout particulièrement préféré.

Les oléfines halogénées engagées dans le procédé selon la présente invention sont en général des dérivés d'un haloéthène ou d'un halopropène, eux-mêmes pouvant être éventuellement substitués. Elles peuvent être substituées par des atomes d'halogène, des groupes alkyle ou halogénoalkyle, des groupes nitrile (CN) ou acide carboxylique (COOH). A titre d'exemples non limitatifs d'oléfines halogénées on peut citer le chlorure de vinyle, le chlorure de vinylidène, le trichloréthylène, les divers isomères des chloropropènes comme le 1-chloroprop-1-ène, le 2-chloroprop-1-ène et le 3-chloroprop-1-ène. Le 2-chloroprop-1-ène convient particulièrement bien.

Le procédé selon l'invention peut être réalisé en continu ou en discontinu. Dans un procédé en discontinu, le rapport molaire entre le composé organique de nickel engagé comme catalyseur et l'oléfine halogénée est habituellement supérieur ou égal à 0,0001. Avantageusement, il est supérieur ou égal à 0,001. De préférence, il est supérieur ou égal à 0,005. Le rapport molaire entre le composé organique du nickel engagé et l'oléfine halogénée est habituellement inférieur ou égal à 1. Avantageusement, il est inférieur ou égal à 0,5. De préférence, il est inférieur ou égal à 0,1.

Dans un procédé en continu, le rapport molaire entre le catalyseur engagé et l'oléfine halogénée évolue approximativement entre les mêmes limites que dans un procédé en discontinu.

Le rapport molaire entre l'haloalcane et l'oléfine halogénée mise en oeuvre peut varier dans de larges mesures. Ce rapport est en général égal ou supérieur à 0,1. Avantageusement, ce rapport est égal ou supérieur à 0,5. D'une manière préférée, il est supérieur ou égal à 1. Généralement, ce rapport est toutefois égal ou inférieur à 20. Avantageusement, ce rapport est égal ou inférieur à 15. D'une manière préférée, ce rapport est égal ou inférieur à 10.

Généralement, la réaction se déroule à une température supérieure ou égale à la température ambiante. De préférence, la température est supérieure ou égale à 30 °C. En général, la température de réaction ne dépasse pas 100°C. De préférence, la température est inférieure ou égale à 70°C.

La durée de la réaction dans un procédé en discontinu ou le temps de séjour dans un procédé en continu sont fonction de divers paramètres tels que la température de réaction, la concentration en réactifs et en catalyseur dans le mélange réactionnel et leurs rapports molaires. En général, en fonction de ces paramètres, le temps de séjour ou la durée de réaction peuvent varier de 5 minutes à 10 heures.

La pression est habituellement supérieure ou égale à la pression atmosphérique et égale ou inférieure à 1.5 MPa (15 bars).

La réaction d'addition est généralement réalisée en phase liquide. Elle peut être réalisée en présence d'un solvant. Avantageusement, le solvant de la réaction est un solvant polaire tel qu'un alcool ou un nitrile. Parmi les alcools utilisables comme solvant pour la réaction, on trouve notamment le méthanol, l'éthanol, l'isopropanol et le tert-butanol. Parmi les nitriles utilisables comme solvant pour la réaction, on trouve les nitriles aliphatiques ou aromatiques. Parmi les nitriles aliphatiques, on trouve notamment l'acétonitrile, le propionitrile ou l'adiponitrile. Parmi les nitriles aromatiques, on trouve notamment le benzonitrile ou le tolunitrile.. Le propionitrile et l'adiponitrile sont préférés. La quantité de solvant engagé dans la réaction n'est pas critique. Toutefois, une solution trop diluée n'est pas favorable à un rendement et à un taux de conversion élevés. De préférence, le rapport molaire du solvant à l'oléfine halogénée est égal ou supérieur à 0,05. Avantageusement, ce rapport est égal ou supérieur à 0,1. Le rapport molaire du solvant à l'oléfine halogénée est en général égal ou inférieur à 30. Avantageusement, il est égal ou inférieur à 20. D'une manière préférée, ce rapport est égal ou supérieur à 1 et égal ou inférieur à 15.

Le procédé de l'invention permet donc de synthétiser des hydrocarbures halogénés en une seule étape, au départ de réactifs facilement accessibles, avec un taux de conversion des réactifs de 75 à 100 %, avec un rendement de 65 à 100 % et une sélectivité de 80 à 100 %.

Les hydrocarbures halogénés obtenus selon le procédé de l'invention sont des précurseurs des analogues fluorés correspondants, lesquels peuvent être facilement obtenus par traitement avec du fluorure d'hydrogène en présence d'un catalyseur tel qu'un sel d'antimoine, un sel de titane, un sel de tantale ou un sel d'étain.

Les exemples ci-après illustrent l'invention de manière non limitative. Dans ces exemples, on a introduit les réactifs, le solvant et le catalyseur dans un réacteur en verre à double enveloppe de 200 ml muni d'une entrée de gaz, surmonté d'un réfrigérant (-78°C) et conditionné sous hélium. Les réactifs sont introduits à la seringue au travers d'un septum. Le chauffage est assuré par une circulation d'huile dans la double enveloppe du réacteur. L'agitation est réalisée par un barreau magnétique. Après refroidissement, un échantillon de liquide est prélevé au moyen d'une seringue et dosé par une méthode chromatographique pour déterminer le taux de conversion de l'oléfine halogénée ainsi que la sélectivité et le rendement en hydrocarbure halogéné.

Dans les exemples ci-dessous, le taux de conversion est le rapport entre la concentration initiale en oléfine halogénée diminuée de sa concentration finale divisée par la concentration initiale, multiplié par 100 ; la sélectivité en hydrocarbure halogéné est le rapport entre la concentration finale en hydrocarbure halogéné divisée par la concentration initiale en oléfine halogénée diminuée de sa concentration finale, multiplié par 100 ; le rendement en hydrocarbure halogéné est le rapport entre la concentration finale en hydrocarbure halogéné divisé par la concentration initiale en oléfine halogénée multiplié par 100.

### Exemple 1 (conforme à l'invention)

Dans cet exemple, on a préparé du 1,1,1,3,3-pentachlorobutane par réaction entre du 2-chloroprop-1-ène, du tétrachlorure de carbone en présence de catalyseur au nickel de formule I dans laquelle R représente de l'hydrogène et X représente un ion bromure et en présence de propionitrile dans les rapports molaires 1/4/0,01/9. La réaction est effectuée à une température de 40°C pendant 3 heures. Le 1,1,1,3,3-pentachlorobutane est obtenu avec un rendement de 67 %, une sélectivité de 86 % et un taux de conversion de l'oléfine halogénée de 78 %.

### Exemple 2 (conforme à l'invention)

Dans cet exemple, on a préparé du 1,1,1,3,3-pentachlorobutane par réaction entre du 2-chloroprop-1-ène, du tétrachlorure de carbone en présence de catalyseur au nickel de formule I dans laquelle R représente de l'hydrogène et X représente un ion bromure et en présence d'adiponitrile dans les rapports molaires 1/4/0,02/6. La réaction est effectuée à une température de 40°C pendant 1 heure. Le 1,1,1,3,3-pentachlorobutane est obtenu avec un rendement de 79 %, une sélectivité de 88 % et un taux de conversion de l'oléfine halogénée de 90 %.

## Revendications

1. Procédé de préparation d'hydrocarbures halogénés comprenant au moins 3 atomes de carbone par réaction entre un haloalcane choisi parmi les composés organiques saturés contenant de un à trois atomes de carbone et au moins 2 atomes de chlore et une oléfine halogénée en présence d'un catalyseur **caractérisé en ce que** le catalyseur est un composé organique du nickel

2. Procédé selon la revendication 1 **caractérisé en ce que** le composé organique du nickel est choisi parmi les sels de nickel (II) formés avec les acides carboxyliques, et parmi les organométalliques dans lesquels le nickel est lié à une atome de carbone aliphatique ou aromatique.

3. Procédé selon la revendication 2 **caractérisé en ce que** le composé organique du nickel est choisi parmi les organométalliques dans lesquels le nickel est lié à un atome de carbone aromatique.

4. Procédé selon la revendication 3 **caractérisé en ce que** le composé organique du nickel répond à la formule générale [Ni^{II}{4-RC₆H₂(CH₂NMe₂)₂₋ 2,6}X] dans laquelle R représente un atome d'hydrogène ou un groupement électrodonneur et X représente un ion halogénure.

5. Procédé selon l'une quelconque des revendications 1 à 4 **caractérisé en ce que** la réaction se déroule en présence d'un solvant.

6. Procédé selon la revendication 5 **caractérisé en ce que** le solvant est un nitrile aliphatique ou aromatique.

7. Procédé selon l'une quelconque des revendications 1 à 6 **caractérisé en ce que** le rapport molaire entre le composé organique du nickel et l'oléfine halogénée est supérieur ou égal à 0,0001 et inférieur ou égal à 1 et le rapport molaire entre l'haloalcane et l'oléfine halogénée est supérieur ou égal à 0,1 et inférieur ou égal à 20.

8. Procédé selon l'une quelconque des revendications 1 à 6 **caractérisé en ce que** l'oléfine halogénée est un dérivé d'un haloéthène ou d'un halopropène.

9. Procédé selon la revendication 8 **caractérisé en ce que** l'hydrocarbure halogéné est le 1,1,1,3,3-pentachlorobutane ou le 1,1,1,3,3-pentachloropropane.

10. Procédé selon l'une quelconque des revendications 1 à 9 **caractérisé en ce que** la réaction se déroule à une température supérieure ou égale à 30 °C et inférieure ou égale à 70 °C.

## Patentansprüche

1. Verfahren zur Herstellung von halogenierten Kohlenwasserstoffen mit wenigstens 3 Kohlenstoffatomen durch Reaktion zwischen einem Halogenalkan, ausgewählt unter den gesättigten organischen Verbindungen mit einem bis drei Kohlenstoffatomen und wenigstens 2 Chloratomen, und einem halogenierten Olefin in Gegenwart eines Katalysators, **dadurch gekennzeichnet, dass** der Katalysator eine organische Nickelverbindung ist.

2. Verfahren gemäß Anspruch 1, **dadurch gekennzeichnet, dass** die organische Nickelverbindung unter den mit den Carbonsäuren gebildeten Nickel (II)-Salzen und unter den metallorganischen Verbindungen, in denen Nickel an ein aliphatisches oder aromatisches Kohlenstoffatom gebunden ist, ausgewählt ist.

3. Verfahren gemäß Anspruch 2, **dadurch gekennzeichnet, dass** die organische Nickelverbindung unter den metallorganischen Verbindungen ausgewählt ist, in denen Nickel an ein aromatisches Kohlenstoffatom gebunden ist.

4. Verfahren gemäß Anspruch 3, **dadurch gekennzeichnet, dass** die organische Nickelverbindung der allgemeinen Formel [Ni^{II}{4-RC₆H₂-2,6-(CH₂NMe₂)₂}X] entspricht, in der R ein Wasserstoffatom oder eine Elektronendonorgruppe darstellt und X ein Halogenidion darstellt.

5. Verfahren gemäß einem der Ansprüche 1 bis 4, **dadurch gekennzeichnet, dass** die Reaktion in Gegenwart eines Lösungsmittels abläuft.

6. Verfahren gemäß Anspruch 5, **dadurch gekennzeichnet, dass** das Lösungsmittel ein aliphatisches oder aromatisches Nitril ist.

7. Verfahren gemäß einem der Ansprüche 1 bis 6, **dadurch gekennzeichnet, dass** das Molverhältnis zwischen der organischen Nickelverbindung und dem halogenierten Olefin größer oder gleich 0,0001 und kleiner oder gleich 1 ist und das Molverhältnis zwischen dem Halogenalkan und dem halogenierten Olefin größer oder gleich 0,1 und kleiner oder gleich 20 ist.

8. Verfahren gemäß einem der Ansprüche 1 bis 6, **dadurch gekennzeichnet, dass** das halogenierte Olefin ein Derivat eines Halogenethens oder eines Halogenpropens ist.

9. Verfahren gemäß Anspruch 8, **dadurch gekennzeichnet, dass** der halogenierte Kohlenwasserstoff 1,1,1,3,3-Pentachlorbutan oder 1,1,1,3,3-Pentachlorpropan ist.

10. Verfahren gemäß einem der Ansprüche 1 bis 9, **dadurch gekennzeichnet, dass** die Reaktion bei einer Temperatur höher oder gleich 30°C und niedriger oder gleich 70°C abläuft.

## Claims

1. Process for the preparation of halohydrocarbons comprising at least 3 carbon atoms, by reaction between a haloalkane selected from the saturated organic compounds containing from 1 to 3 carbon atoms and at least 2 chlorine atoms and a haloolefin in the presence of a catalyst, **characterized in that** the catalyst is an organonickel compound.

2. Process according to Claim 1, **characterized in that** the organonickel compound is chosen from nickel(II) salts formed with carboxylic acids, and from organometallic compounds in which the nickel is attached to an aliphatic or aromatic carbon atom.

3. Process according to Claim 2, **characterized in that** the organonickel compound is chosen from organometallic compounds in which the nickel is attached to an aromatic carbon atom.

4. Process according to Claim 3, **characterized in that** the organonickel compound corresponds to the general formula [Ni^{II}{4-RC₆H₂(CH₂NMe₂)₂-2,6}X] in which R represents a hydrogen atom or an electron-donating group and X represents a halide ion.

5. Process according to any one of Claims 1 to 4, **characterized in that** the reaction takes place in the presence of a solvent.

6. Process according to Claim 5, **characterized in that** the solvent is an aliphatic or aromatic nitrile.

7. Process according to any one of Claims 1 to 6, **characterized in that** the molar ratio between the organonickel compound and the haloolefin is greater than or equal to 0.0001 and less than or equal to 1, and the molar ratio between the haloalkane and the haloolefin is greater than or equal to 0.1 and less than or equal to 20.

8. Process according to any one of Claims 1 to 6, **characterized in that** haloolefin is a haloethene or halopropene derivative.

9. Process according to Claim 8, **characterized in that** the halohydrocarbon is 1,1,1,3,3-pentachlorobutane or 1,1,1,3,3-pentachloropropane.

10. Process according to any one of Claims 1 to 9, **characterized in that** the reaction takes place at a temperature greater than 30°C and less than 70°C.
